# FASCICULE DE BREVET EUROPEEN

(11) **EP 1 633 375 B1**
(45) Date de publication et mention de la délivrance du brevet: **23.11.2011**
(21) Numéro de dépôt: 04767183.9
(22) Date de dépôt: 26.05.2004
(51) Int. Cl.: A61K 33/06, A61K 33/08, A61K 33/14

(54) **COMBINAISON D'UNE PREPARATION DE MAGNESIUM A LIBERATION PROLONGEE ET DE CERTAINS EXTRAITS DE PLANTES, UTILISATION DANS LE DOMAINE COSMETIQUE, THERAPEUTIQUE ET/OU NUTRITIONEL**
KOMBINATION AUS EINEM MAGNESIUMPRÄPARAT MIT VERLÄNGERTER FREISETZUNG UND SPEZIFISCHEN PFLANZENEXTRAKTEN UND VERWENDUNG IN DEN BEREICHEN KOSMETIK, THERAPEUTIKA UND/ODER ERNÄHRUNG
COMBINATION CONSISTING OF A MAGNESIUM PREPARATION HAVING A PROLONGED RELEASE AND OF CERTAIN PLANT EXTRACTS, AND USE THEREOF IN THE FIELDS OF COSMETICS, THERAPEUTICS AND/OR NUTRITION

(30) Priorité: 27.05.2003 FR 0306411
(43) Date de publication de la demande: 15.03.2006
(73) Titulaire: Joanny Menvielle-Bourg, Fabienne, 75008 Paris (FR)
(72) Inventeur: Joanny Menvielle-Bourg, Fabienne, 75008 Paris (FR)
(74) Mandataire: Clisci, Serge
(86) Numéro de dépôt international: PCT/FR2004/001305
(87) Numéro de publication internationale: WO 2004/105778

(56) Documents cités:
- FR-A- 2 296 426
- FR-A- 2 616 068
- FR-A- 2 677 546
- US-A- 5 976 568

## Description

### Domaine de l'invention

La présente invention a trait à une nouvelle combinaison mettant en oeuvre une préparation (I) à base de Mg à libération prolongée (i.e. « Mg retard »), d'une part, et au moins une substance active (Z) définie ci-après, d'autre part, ladite combinaison étant utile dans le domaine cosmétique, thérapeutique et/ou nutritionnel. Cette nouvelle combinaison est particulièrement intéressante en cosmétique et dermopharmacie vis-à-vis des états de stress, et dans le domaine de la nutrition en tant que complément nutritionnel. Cette combinaison I+Z peut être formulée soit sous un même conditionnement, soit sous des conditionnements distincts ou séparés dans le cadre d'un polytraitement médicamenteux, nutritionnel et/ou cosmétique.

### Art antérieur

L'art antérieur le plus proche est constitué par le brevet européen délivré EP 0542979 B. Ce brevet visé une compositions thérapeutique utile vis-à-vis des carences magnésiques et destinée à assurer la libération du magnésium, qu'elle contient, de façon lente et continue sous forme de Mg²⁺ assimilable au niveau entéral afin de compléter jusqu'à au moins 6mg/kg l'apport quotidien en magnésium chez l'homme, ladite composition étant caractérisée en ce qu'elle comporte un mélange comprenant, en association avec un excipient physiologiquement acceptable,
(a) 4 à 14 parties en poids de magnésium provenant d'une source constituée par MgO, MgCl₂ et les hydrates de chlorure de magnésium de formule MgCl₂.n(H₂O) où n est un nombre entier ou fractionnaire supérieur à 0 et inférieur ou égal à 6,
(b) 6 à 13 parties en poids d'un polymère hydrophile , quand la source de Mg est MgO, ou 10 à 30 parties en poids d'une substance hydrophobe choisie parmi l'ensemble constitué par les polymères hydrophobes physiologiquement acceptables , les esters d'acide gras et leurs mélanges, quand la source de Mg est différente de MgO, et
(c) 6 à 16 parties en poids d'une charge inerte intervenant en tant que diluant solide et notamment choisie parmi l'ensemble constitué par le lactose, les phosphates de métaux alcalins et alcalino-terreux et leurs mélanges,
la teneur en Mg dans ledit mélange étant comprise entre 5 et 60 % en poids par rapport au poids dudit mélange."

La solution technique fournie par le brevet EP 0542979 B est certes très efficace. Cependant on constate que (i) l'utilisation par voie orale dans un même noyau d'un polymère hydrophobe (tel que le PVC) et MgCl₂.n(H₂O) et (ii) celle d'un polymère hydrophile (tel que la PVP) et MgO, soulèvent des difficultés eu égard aux réglementations de certains états membres de l'Union Européenne.

Pour pallier ces difficultés, l'on se propose de fournir une nouvelle solution technique, qui se distingue de l'enseignement du brevet antérieur par la suppression des PVC et PVP, d'une part, et surtout la combinaison de la composition de Mg à libération prolongée avec au moins une autre substance active (Z), d'autre part.

On a en effet trouvé de façon surprenante qu'une telle combinaison présente une synergie par rapport au magnésium [fourni sous la forme précitée de Mg0, MgCl₂ ou MgCl₂.n(H₂0)] et à Z, quand la préparation I et la substance Z sont administrées séparément ou sous un même conditionnement.

### Objet de l'invention

Selon un premier aspect de l'invention, l'on fournit une nouvelle combinaison vis-à-vis des carences en magnésium, qui est utile notamment
(i) en thérapeutique, en tant que médicament,
(ii) en cosmétique, et/ou
(iii) dans le domaine de la nutrition, en tant que complément nutritionnel.

Plus précisément, on préconise selon l'invention une nouvelle combinaison utile en cosmétique, en thérapeutique et/ou dans le domaine de la nutrition, pour agir notamment contre les états de stress, caractérisée en ce qu'elle consiste en
(α) une préparation (I) de magnésium à libération prolongée comprenant, en association avec un excipient physiologiquement acceptable, un mélange :
   (A) de magnésium provenant d'une source de magnésium constituée par MgO, MgCl₂ et les hydrates de formule MgCl₂.nH₂O où n est un nombre entier ou fractionnaire supérieur à 0 ou égal à 6,
   (B) d'au moins une substance choisie parmi B1, B2 ou une de leurs associations :
      (B1) un polymère hydrophile, qui est un dérivé de cellulose, et/ou
      (B2) une substance hydrophobe faisant partie de la famille des esters d'acide gras avec des polyols, et
   (C) d'une charge inerte intervenant en tant que diluant, notamment le lactose,
   la teneur en magnésium étant comprise entre 1 et 60 %, en poids par rapport au poids dudit mélange A + B + C ; et,
(β) une substance active (Z), qui est un extrait naturel de plante. Parmi les substances Z, **on choisit parmi** : un extrait d'aubépine, un extrait de valériane, un extrait de mélisse, un extrait de thym maritime, un extrait d'écorce de pin maritime, un extrait d'aubier de tilleul, un un hydrolysat de protéines de blé et/ou de riz, un extrait de soja, un extrait de pomme, un extrait de melon, un extrait de papaye, un extrait d'ananas, ou un de leurs mélanges

Selon l'invention, on préconise également l'utilisation de la présente combinaison pour compléter l'apport quotidien en magnésium, dans le domaine cosmétique, dans le domaine dermatothérapeutique et/ou dans le domaine de la nutrition.

On préconise également l'utilisation de la préparation (I) et de ladite substance (Z), pour l'obtention d'un médicament destiné au traitement des carences en magnésium.

### Description détaillée de l'invention

De façon avantageuse, ledit mélange de A, B et C forme un noyau à libération prolongée qui est susceptible d'être logé à l'intérieur d'un enrobage gastrorésistant du type pelliculage.

Le polymère hydrophile B1 est avantageusement choisi parmi des polymères cellulosiques dérivés de la cellulose : notamment la carboxy-méthylcellulose, l'hydroxypropylcellulose, l'hydroxypropylméthylcellulose et leurs mélanges.

La substance hydrophobe B2 est avantageusement un ester d'acide gras, où ledit acide gras est en C₈-C₂₄ et le reste du composant alcool comporte au moins un reste polyol (tel que glycérol et/ou polyéthylèneglycol). Ladite substance B2 peut être un mélange d'esters d'acides gras. En particulier, la substance hydrophobe peut être le palmitate de glycérol et/ou le béhénate de glycérol.

La charge inerte (C) est avantageusement le lactose (de préférence le lactose anhydre).

Le mélange A + B + C peut contenir d'autres additifs usuels dans l'art de la galénique, notamment de la silice (silice colloïdale) et/ou un lubrifiant. La teneur en magnésium dans ledit mélange A + B + C sera comprise entre 1 et 60% en poids.

L'enrobage gastrorésistant est, comme indiqué plus haut, avantageusement un pelliculage. Il est plus particulièrement formé de monoglycérides acétylés et de gomme laque ou encore d'acétophtalate de cellulose, d'un mélange acétophtalate de cellulose/polyéthylèneglycol, d'un mélange acétophtalate de cellulose/phtalate d'alkyle en C₁-C₅, d'un mélange acétophtalate de cellulose/polyéthylèneglycol/phtalate d'alkyle en C₁-C₅.

De façon pratique, dans ladite préparation (I) :
le rapport pondéral B/A sera compris entre 0,8/1 et 8,2/1 (de préférence entre 1,2/1 et 4,8/1),
le rapport pondéral C/A sera compris entre 0,4/1 et 4/1 (de préférence entre 0,5/1 et 2,3/1), et
la quantité de Mg par unité galénique, notamment sous la forme d'un comprimé, variera entre 10 mg/unité galénique à 250 mg/unité galénique, les dosages préférés en Mg étant de 30 mg/unité galénique, 50 mg/unité galénique ou 100 mg/unité galénique.

De façon avantageuse, la teneur en Mg dans ladite préparation I se situera entre 1 et 60 % en poids par rapport au poids de ladite préparation 1. On prévoit une administration d'un seul produit *per os* constitué de I + Z (i. e. un seul conditionnement), la ou les substances actives Z étant alors incorporées :
(i) dans le noyau contenant A, B et C,
(ii) dans l'enveloppe de l'enrobage gastrorésistant de ladite préparation (I),
(iii) dans une couche non gastrorésistante entourant le noyau contenant A, B et C, ou
(iv) dans une couche entourant ledit enrobage gastrorésistant.

Dans le cas d'une administration sous un seul conditionnement, la substance Z peut être ajoutée à la source de magnésium ou est susceptible de remplacer une portion du magnésium fourni par ladite source.

Il est intéressant de pouvoir disposer d'une couche non gastrorésistante autour de la préparation I ou autour de l'enveloppe gastroresistante, quand on a besoin de libérer rapidement une substance Z qu'elle contient.

D'un point de vue pratique, parmi les extraits de plante on fera appel aux extraits dits officinaux qui sont préparés conformément à la pharmacopée. Pour les extraits de céréales (telles que le blé et le riz) et de soja, on pourra utiliser selon l'invention des hydrolysats de protéines végétales ; par exemple l'hydrolysat de gluten de blé ou l'hydrolysat de protéine de riz, qui contiennent chacun des peptides et des aminoacides.

On recommande également des extraits de fruit, par exemple l'extrait de papaye éventuellement fermenté, l'extrait officinal d'ananas, qui est riche en bromélaine, et l'extrait de melon, qui est riche en superoxyde dismutase (SOD). . On recommande plus particulièrement l'extrait de pomme qui est riche en ω³.
les substances Z, on peut également ajouter une ou plusieurs vitamines (notamment la vitamine B12, la vitamine E et/ou la vitamine D) et un ou plusieurs oligoéléments ou minéraux (notamment Zn, Mn, Cu).

Dans le domaine cosmétique, dermatopharmaceutique et nutritionnel,
(i) vis-à-vis du stress et du vieillissement au niveau du derme et de l'épiderme, l'on recommande d'associer la préparation I et l'extrait de mélisse, sous un même conditionnement oral,
(ii) vis-à-vis des troubles associés à la ménaupause, l'on recommande d'associer la préparation I et un extrait de soja contenant de la daidzine et/ou de la génistine, et vis-à-vis des agents oxydants un extrait de pin maritime riche en proanthocyanidols, sous un même conditionnement oral.

D'autres avantages et caractéristiques seront mieux compris à la lecture qui va suivre d'exemples de réalisation nullement limitatifs.

### Exemple 1

### Comprimés à libération prolongée dosés à 100 mg de magnésium

On a préparé des comprimés pelliculés selon le procédé de l'exemple 1 de EP 0542979 B en remplaçant MgO par MgCl₂.6H₂O, chaque comprimé ayant

### un noyau contenant :

| | | |
|---|---|---|
| MgCl₂,6H₂O | 836,45 mg | |
| lactose anhydre (diluant) | 52,64 mg | C/A = 0,52/1 |
| silice colloïdale hydratée (agent liant) | 13,03 mg | |
| hydroxypropylméthylcellulose (gélifiant) | 120,00 mg | |
| palmitate de glycérol (lubrifiant) | 112,88 mg | B/A = 2,32/1 |

et un pelliculage.

### Exemple 2

### Comprimés à libération prolongée dosés à 50 mg de magnésium

On a préparé des comprimés pelliculés comme indiqué ci-dessus, chaque comprimé ayant

### un noyau contenant :

| | | |
|---|---|---|
| MgCl₂,6H₂O | 418,22 mg | |
| lactose anhydre (diluant) | 27,38 mg | C/A = 0,54/1 |
| silice colloïdale hydratée (agent liant) | 10,00 mg | |
| hydroxypropylméthylcellulose (gélifiant) | 55,00 mg | |
| palmitate de glycérol (lubrifiant) | 61,40 mg | B/A = 2,33/1 |

et un enrobage gastrorésistant.

### Exemple 3

### Comprimés à libération prolongée dosés à 30 mg de magnésium et 40 mg d'aubépine

On a préparé des comprimés pelliculés comme indiqué ci-dessus, chaque comprimé ayant

### un noyau contenant :

| | | |
|---|---|---|
| MgCl₂,6H₂O | 250,93 mg | |
| extrait officinal d'aubépine | 40,00 mg | |
| lactose anhydre (diluant) | 27,38 mg | C/A = 0,91/1 |
| silice colloïdale hydratée (agent liant) | 10,00 mg | |
| hydroxypropylméthylcellulose (gélifiant) | 55,00 mg | |
| palmitostéarate de glycérol (lubrifiant) | 61,49 mg | B/A = 3,88/1 |

et un pelliculage.

### Exemple 4

### Comprimés retard dosés à 50 mg de magnésium et 50 mg d'aubépine

On a préparé des comprimés pelliculés comme indiqué ci-dessus, chaque comprimé ayant

### un noyau contenant :

| | | |
|---|---|---|
| MgCl₂,6H₂O | 418,22 mg | |
| extrait officinal d'aubépine | 50,00 mg | |
| lactose anhydre (diluant) | 52,64 mg | C/A =1,10/1 |
| silice colloïdale hydratée (agent liant) | 13,03 mg | |
| hydroxypropylméthylcellulose (gélifiant) | 120,00 mg | |
| palmitate de glycérol (lubrifiant) | 112,88 mg | B/A = 4,65/1 |

et un enrobage gastrorésistant.

### Exemple 5

### Comprimés à libération prolongée dosés à 100 mg de magnésium plus hydrolysat de protéines de blé

On a préparé des comprimés comme indiqué ci-dessus, chaque comprimé ayant

### un noyau contenant :

| | |
|---|---|
| MgO | 165,84 mg |
| Hydrosat de gluten de blé (commercialisé sous la nomenclature | 116,16 mg |
| de HYPROBLE^{®} et contenant 27 à 32 % en poids de peptides/protéines et moins de 5 % en poids d'aminoacides libres) lactose anhydre | 50,00 mg |
| silice colloïdale | 13,03 mg |
| hydroxypropylméthylcellulose | 110,00 mg |
| mono- et distéarate de glycérol | 80,77 mg |
| stéarate de magnésium | 12,20 mg |

et un enrobage gastrorésistant
à base de gomme laque.

### Exemple 6

### Comprimés à libération prolongée dosés à 100 mg de magnésium plus autres ingrédients

On a préparé des comprimés comme indiqué ci-dessus, chaque comprimé ayant

### un noyau contenant :

| | |
|---|---|
| HYPROMAG^{®} (mélange de MgO marin, fournissant 100 mg de Mg, et 118,16 mg d'hydrolysat de protéine de riz) | 286 mg |
| extrait officinal d'aubépine | 30 mg |
| extrait officinal de pavot de Californie | 10 mg |
| lactose anhydre | 50,00 mg |
| silice colloïdale | 13,03 mg |
| hydroxypropylméthylcellulose | 110,00 mg |
| mono- et distéarate de glycérol | 80,77 mg |
| stéarate de magnésium | 12,20 mg |

et un enrobage gastrorésistant
à base de gomme laque.

On obtient un produit final particulièrement efficace contre le stress et l'anxiété légère à modérée.

### Exemple 9

On prépare un comprimé selon l'exemple 5 ci-dessus, sans son enrobage gastrorésistant. On dépose à la surface de ce comprimé une couche polymérique (du type polyacrylate/polyméthacrylate) contenant un extrait d'écorce de pin maritime. Après séchage, on peut, le cas échéant, enrober le produit résultant à l'intérieur d'un enrobage gastrorésistant.

### Essais de dissolution

Les compositions selon l'invention présentent un profil de dissolution satisfaisant, déterminé dans un essai de dissolution *in vitro* dans les conditions suivantes :
- Cinétique de dissolution in vitro sur 8 heures, en milieu tampon pH 6,8 avec des prélèvements à 1 heure, 2 heures, 4 heures et 8 heures (à partir de comprimés restés 2 heures en milieu acide chlorhydrique 0,1N) ; et
- Dosage du magnésium par spectrométrie d'absorption atomique.

A titre d'exemple non limitatif, un essai de dissolution peut être mis en oeuvre de la manière suivante :

On utilise un appareil à palettes SOTAX AT7^{®}, en opérant à une température de 37 ± 0,5°C. Une solution tampon pH=6,8 est préparée à partir de 17 g de phosphate monopotassique dans 5000 ml d'eau et ajustement au pH=6,8 au moyen d'une solution de soude 1N. Le volume de dissolution est de 1000 ml de tampon pH=6,8 dans chaque réacteur. La vitesse de rotation des palettes est fixée à 100 t/mn.

L'essai a été mis en oeuvre au moyen d'un comprimé tel que décrit ci-dessus et comportant un enrobage gastrorésistant.

Le dosage du magnésium par absorption atomique est réalisé sur un prélèvement d'échantillon de 1 ml. L'échantillon de 1 ml est dilué à 100 ml dans de l'eau additionnée de 5 ml d'une solution de chlorure de strontium à 100 g/l. La courbe du profil de dissolution est donnée en annexe (figure 1/1).

On constate une première phase jusqu'à la 2ème heure, dans laquelle une faible quantité de magnésium est libérée ; puis une seconde phase de 2 à 4 heures, dans laquelle la plus grande partie du magnésium est libérée ; et enfin une troisième phase entre la 4ème et la 8ème heure, où le reste du magnésium est libéré.

Le profil de dissolution du comprimé étudié atteste de la libération progressive du magnésium et de l'efficacité de la formulation pour couvrir les besoins en magnésium pendant une durée supérieure à 8 heures.

Dans un autre essai de dissolution, le magnésium dissous est également dosé par spectrophotométrie d'absorption atomique. On utilise un spectrophotomètre de flamme UNICAM^{®} PU 9200 X. La solution à tester est diluée 100 fois avec de l'eau Inflac B.Braun.

On effectue des prélèvements de la solution à tester, respectivement de 2,5 ml, 5 ml et 10 ml et le volume est amené à 100 ml au moyen d'une solution de chlorure de césium à 0,2% dans l'acide nitrique 0,5M. Des prélèvements de 25 ml de milieu de dissolution sont effectués après 1 heure et 2 heures puis dilués à 50 ml par de l'eau Inflac. Les solutions sont filtrées et 5 ml de chaque filtrat sont dilués à 100 ml avec une solution de chlorure de césium à 0,2% dans l'acide nitrique 0,5M.

D'autres prélèvements de 25 ml de milieu de dissolution sont effectués après 4 heures et 8 heures puis dilués à 50 ml par de l'eau Inflac. Les solutions sont filtrées et 1 ml de chaque filtrat est dilué à 100 ml avec une solution de chlorure de césium à 0,2% dans l'acide nitrique 0,5M. On détermine la concentration en magnésium de chacune des 4 solutions.

On constate que, après 4 heures, 80% du magnésium présent sont libérés et que, après 8 heures, 95% du magnésium sont libérés.

Les avantages énoncés précédemment se vérifient également dans cet essai.

Les compositions des exemples 2, 3 et 4 donnent des résultats analogues dans les essais de dissolution décrits ci-dessus.

### Essais de résistance antiradicalaire

Des expérimentations de résistance à l'action de radicaux libres générés *in situ* à partir d'un générateur de radicaux libres ont été entrepris (i) sur des hématies de rat recevant les comprimés ou combinaisons de l'invention, et (ii) sur des cultures de cornéocytes humains préalablement mis en contact avec les composants dilués des comprimés et combinaisons selon l'invention, suivant les modalités opératoires décrites dans le brevet européen EP 0418335 B.

On détermine la résistance au stress par la mesure de la résistance libre par le temps de demi-lyse (T_{1/2}) des cellules soumises à l'action du champ de radicaux libres. Les résultats obtenus mettent en évidence la synergie de la solution technique fournie par l'invention vis-à-vis de la somme (obtenue par le calcul) des effets de Mg administré seul et de la substance Z administrée seule.

## Revendications

1. Combinaison utile en cosmétique, en thérapeutique et/ou dans le domaine de la nutrition, pour agir notamment contre les états de stress, **caractérisée en ce qu'**elle consiste en
(α) une préparation (I) de magnésium à libération prolongée comprenant, en association avec un excipient physiologiquement acceptable un mélange :
(A) de magnésium provenant d'une source de magnésium constituée par MgO, MgCl₂ et les hydrates de formule MgCl₂.nH₂O où n est un nombre entier ou fractionnaire supérieur à 0 ou égal à 6,
(B) d'au moins une substance choisie parmi B1, B2 ou une de leurs associations :
(B1) un polymère hydrophile, qui est un dérivé de cellulose, et/ou
(B2) une substance hydrophobe faisant partie de la famille des esters d'acide gras avec des polyols, et
(C) d'une charge inerte intervenant en tant que diluant, notamment le lactose,
la teneur en magnésium étant comprise entre 1 et 60 %, en poids par rapport au poids dudit mélange A + B + C ; et,
(β) une substance active (Z), qui est un extrait naturel de plante choisi parmi l'ensemble constitué par un extrait d'aubépine, un extrait de valériane, un extrait de mélisse, un extrait de thym maritime, un extrait d'écorce de pin maritime, un extrait d'aubier de tilleul, un hydrolysat de protéines de blé et/ou de riz, un extrait de soja, un extrait de pomme, un extrait de melon, un extrait de papaye, un extrait d'ananas ou un de leurs mélanges.

2. Combinaison suivant la revendication 1, **caractérisée en ce que** ledit mélange de A, B et C forme un noyau à libération prolongée qui est susceptible d'être logé à l'intérieur d'un enrobage gastrorésistant du type pelliculage.

3. Combinaison à base de magnésium suivant la revendication 1 ou 2, **caractérisée en ce que** ladite substance active (Z) est susceptible de remplacer une portion du magnésium contenu dans ladite préparation (I).

4. Combinaison selon les revendications 1 et 2, **caractérisée en ce que** ladite préparation (I) et ladite substance active (Z) sont sous le même conditionnement galénique.

5. Combinaison selon la revendication 4, **caractérisée en ce que** ladite préparation (I) et ladite substance active (Z) sont sous un même conditionnement, ladite substance active (Z) étant incorporée
(i) dans le noyau contenant A, B et C,
(ii) dans l'enveloppe de l'enrobage gastrorésistant de ladite préparation (I),
(iii) dans une couche non gastrorésistante entourant le noyau contenant A, B et C, ou
(iv) dans une couche entourant ledit enrobage gastrorésistant.

6. Combinaison selon une quelconque des revendications 1 à 5, **caractérisée en ce que** dans ladite préparation (I) :
le rapport pondéral B/A est compris entre 0,8/1 et 8,2/1 (de préférence entre 1,2/1 et 4,8/1),
le rapport pondéral C/A est compris entre 0,4/1 et 4/1 (de préférence entre 0,5/1 et 2,3/1), et
la quantité de Mg par unité galénique, notamment sous la forme d'un comprimé, varie entre 10 mg/unité galénique à 250 mg/unité galénique, les dosages préférés en Mg étant de 30 mg/unité galénique, 50 mg/unité galénique ou 100 mg/unité galénique.

7. Utilisation de la combinaison selon une quelconque des revendications 1 à 6 dans le domaine cosmétique et/ou dans le domaine de la nutrition pour compléter l'apport quotidien de magnésium.

8. Combinaison selon une quelconque des revendications 1 à 6 pour son utilisation dans le domaine dermatothérapeutique.

## Claims

1. Combination suitable for use in cosmetics, therapeutics and/or in the field of nutrition, particularly to combat states of stress, **characterised in that** it consists of
(α) a sustained-release magnesium preparation (I) comprising, in association with a physiologically acceptable excipient, a mixture:
(A) of magnesium from a source of magnesium consisting of MgO, MgCl₂ and hydrates having a formula MgCl₂.nH₂O where n is an integer or fraction greater than or equal to 6,
(b) at least one substance selected from B1, B2 or any of the associations thereof:
(B1) a hydrophilic polymer, which is a cellulose derivative, and/or
(B2) a hydrophobic substance belonging to the family of fatty acid esters with polyols, and
(c) an inert filler used as a diluent, particularly lactose,
the magnesium content being between 1 and 60%, by weight with respect to the weight of said mixture A + B + C; and
(β) an active substance (Z), which is a natural plant extract selected from the set consisting of hawthorn extract, valerian extract, lemon balm extract, maritime thyme extract, maritime pine bark extract, lime tree sapwood extract, wheat and/or rice protein hydrolysate, soy extract, apple extract, melon extract, papaya extract, pineapple extract or any of the mixtures thereof.

2. Combination according to claim 1, **characterised in that** said mixture of A, B and C forms a sustained-release core suitable for being housed inside a film-coating type gastro-resistant coating.

3. Combination based on magnesium according to claim 1 or 2, **characterised in that** said active substance (Z) is suitable for replacing a portion of the magnesium contained in said preparation (I).

4. Combination according to claims 1 and 2, **characterised in that** said preparation (I) and said active substance (Z) are in the same pharmaceutical form.

5. Combination according to claim 4, **characterised in that** said preparation (I) and said active substance (Z) are in the same pharmaceutical form, said active substance (Z) being incorporated
(i) in the core containing A, B and C
(ii) in the gastro-resistant coating shell of said preparation (I),
(iii) in the non-gastro-resistant layer surrounding the core containing A, B and C, or
(iv) in a layer surrounding said gastro-resistant coating.

6. Combination according to any of claims 1 to 5, **characterised in that**, in said preparation (I):
the ratio B:A by weight is between 0.8:1 and 8.2:1 (preferably between 1.2:1 and 4.8:1),
the ratio C:A by weight between 0.4:1 and 4:1 (preferably between 0.5:1 and 2.3:1), and
the quantity of Mg per dosage unit, particularly in tablet form, varies between 10 mg/dosage unit and 250 mg/dosage unit, the preferred Mg dosages being 30 mg/dosage unit, 50 mg/dosage unit or 100 mg/dosage unit.

7. Use of the combination according to any of claims 1 to 6 in the field of cosmetics and/or in the field of nutrition for supplementing daily magnesium intake.

8. Combination according to any of claims 1 to 6 for the use thereof in the field of dermatotherapeutics.

## Patentansprüche

1. Kombination, verwendbar in der Kosmetik, in der Therapeutik und/oder auf dem Gebiet der Ernährung, zum Wirken insbesondere gegen Stressbedingungen, **dadurch gekennzeichnet, daß** sie besteht aus
(α) einer Magnesiumformulierung mit verzögerter Freisetzung (I), umfassend, in Verbindung mit einem physiologisch verträglichen Exzipienten, ein Gemisch von
(A) Magnesium, abgeleitet von einer Magnesiumquelle, bestehend aus MgO, MgCl₂ und den Hydraten der Formel MgCl₂.nH₂O, wobei n eine ganze Zahl oder ein Bruchteil von größer als 0 oder gleich 6 ist,
(B) mindestens einer Substanz, ausgewählt aus B1, B2 oder einer Kombination davon:
(B1) ein hydrophiles Polymer, welches ein Zellulosederivat ist, und/oder
(B2) eine hydrophobe Substanz, welche zu der Familie der Fettsäureester mit Polyolen gehört, und
(C) einem inerten Füllmittel, welches als Verdünnungsmittel wirkt, insbesondere Lactose,
wobei der Magnesiumgehalt zwischen 1 und 60 Gew.-% relativ zu dem Gewicht des Gemisches A + B + C ist, und
(β) eine aktive Substanz (Z), welche ein natürlicher Pflanzenextrakt ist, ausgewählt aus der Gruppe, bestehend aus Hagedornextrakt, Baldrianextrakt, Melisseextrakt, Thymianextrakt, Seekieferrindenextrakt, Linden-Splintholzextrakt, Weizen- und/oder Reisproteinhydrolysat, Sojaextrakt, Apfelextrakt, Melonenextrakt, Papayaextrakt, Ananas-extrakt oder deren Gemische.

2. Kombination gemäß Anspruch 1, **dadurch gekennzeichnet, dass** das Gemisch von A, B und C einen Kern mit verzögerter Freisetzung bildet, welcher befähigt ist, in einem gastroresistenten Überzug vom Filmbeschichtungstyp eingerichtet zu werden.

3. Kombination auf Magnesium-Basis gemäß Anspruch 1 oder 2, **dadurch gekennzeichnet, dass** die aktive Substanz (Z) befähigt ist, einen Anteil des in der Formulierung (I) enthaltenen Magnesiums zu ersetzen.

4. Kombination gemäß Anspruch 1 oder 2, **dadurch gekennzeichnet, dass** die Formulierung (I) und die aktive Substanz (Z) in der gleichen Dosierungsform sind.

5. Kombination gemäß Anspruch 4, **dadurch gekennzeichnet, dass** die Formulierung (I) und die aktive Substanz (Z) in der gleichen Form sind, mit der aktiven Substanz (Z) eingebracht
(i) in den Kern, enthaltend A, B und C,
(ii) in die Schale des gastroresistenten Überzugs der Formulierung (I),
(iii) in eine nicht-gastroresistente Schicht, welche den Kern, enthaltend A, B und C umgibt, oder
(iv) in eine Schicht, welche die gastroresistente Beschichtung umgibt.

6. Kombination gemäß einem der Ansprüche 1 bis 5, **dadurch gekennzeichnet, dass** in der Formulierung (I) das Gewichtsverhältnis von B/A zwischen 0,8/1 und 8,2/1 (vorzugsweise zwischen 1,2/1 und 4,8/1) ist,
das Gewichtsverhältnis von C/A zwischen 0,4/1 und 4/1 (vorzugsweise zwischen 0,5/1 und 2,3/1) ist und
die Menge an Mg pro Dosierungseinheit, insbesondere in der Form einer Tablette, zwischen 10 mg/Dosierungseinheit und 250 mg/Dosierungseinheit variiert, wobei die bevorzugten Dosierungen an Mg 30 mg/Dosierungseinheit, 50 mg/Dosierungseinheit oder 100 mg/Dosierungseinheit sind.

7. Verwendung der Kombination gemäß einem der Ansprüche 1 bis 6 im Bereich der Kosmetik und/oder im Bereich der Ernährung, um den Tagesbedarf an Magnesium zu ergänzen.

8. Kombination gemäß einem der Ansprüche 1 bis 6 zur Verwendung im Bereich der Dermatherapeutik.
